# EUROPEAN PATENT APPLICATION

(11) **EP 4 603 134 A1**
(43) Date of publication of application: **20.08.2025**
(21) Application number: 24158170.1
(22) Date of filing: 16.02.2024
(51) Int. Cl.: A61N 1/04, A61N 1/36

(54) **METHOD FOR SETTING UP A STIMULATION ELECTRODE CONTROL SYSTEM**

(71) Applicant: Exoneural Network AB, 17073 Solna (SE)
(72) Inventor: ABBOUD, Nicolas Loren, 1110 Wien (AT)
(74) Representative: Gramm, Lins & Partner Patent- und Rechtsanwälte PartGmbB

(57) **Abstract**

The invention relates to a method for setting up a control system for stimulation electrodes (4) which are arranged on at least one garment (2) with at least one textile component (28) and are coupled to a control device in which an activation program is stored, with which it is determined when and which stimulation electrode (4) is electrically activated, deactivated or modulated, characterised in that at least one basic program is stored in the activation program, in that at least one parameter individual to the user of the garment (2) is entered for each basic program and in that the basic program is automatically changed and a stimulation program or training program is created as a function of the at least one parameter.

## Description

The invention relates to a method for setting up a control system for stimulation electrodes which are arranged on at least one garment with at least one textile component or textile surface and are coupled to a control device in which an activation program is stored which is used to determine when which electrode is electrically activated, deactivated or modulated.

Data can be recorded and muscles and/or nerves can be stimulated in different ways. Sensors and/or electrodes can be arranged on the body in various ways for this purpose. Both the purpose of the arrangement of the electrodes and/or sensors and the way in which they are arranged are varied.

A vest for providing electrical stimulation is known from US 7 072 721 B1. The vest comprises front and back areas and a controller. The electrodes are arranged on the interior surfaces of the right and left front areas and the back area. The controller is arranged on the exterior surface of the front area and provides adjustable electrical pulse signals for the electrodes via wires. The vest can be used for electrostimulation of muscles or nerves of the abdomen and back of a user.

US 2002/0077688 A1 discloses a garment made of a flexible, elastic material that adapts to the user's body. A device for the electrical stimulation of muscles and/or nerves is arranged on the garment. The garment is designed in particular to position the electrodes at predetermined positions on the body, the positions corresponding to different muscles or nerve groups. Wires connect the electrodes to an interface connector to connect the garment to an external controller of the stimulation device. Via the controller, it is possible to send stimulation signals to one or more electrodes to address specific muscles or nerve groups of the body. The connection to the external device is made via a plug.

US 2012/0 245 483 A1 discloses a system and a garment for relaxing spastic muscles. The system causes muscle relaxation by reducing the muscular spasticity through the stimulation of joints and muscles and consists of a garment with electrodes, a hardware unit and software that controls the stimulation.

US 2020/0078583 A1 discloses an elasticated garment that is designed to fit snugly on the user's body. The garment is designed for electrostimulation therapy of muscles and has electrodes on an inner surface of the garment. The electrodes are in contact with the user's skin. The garment comprises electrical connections designed to connect the electrodes to one or more connection units. The connection units are integrated into the fabric and have, for example, a flat extension and are flexible. Magnets can be used to attach a connection pad to the connection unit.

It is often difficult to set up a control unit for garments with several electrodes. For this reason, a control program is permanently stored in a control unit and retained over a longer period of time. The control program is created by an orthopaedic technician and is customised to the individual patient.

The purpose of the present invention is to provide a method for facilitating the individualised control of stimulation electrodes.

This problem is solved by a method with the features of the main claim. Advantageous embodiments and further embodiments of the invention are disclosed in the dependent claims, the description and the figures.

The method for setting up a control system for stimulation electrodes which are arranged on at least one garment with at least one textile component or textile surface and are coupled to a control device in which an activation program is stored, with which it is determined when which electrode is electrically activated, deactivated or modulated, is characterised in that that a basic program is initially stored in the activation program, whereby at least one parameter individual to the user of the garment is entered for each basic program and that the basic program is automatically changed as a function of the at least one parameter and a stimulation program, in particular a training program, is created. The stimulation via surface electrodes arranged on the inside or interior surface of a garment is used, for example, to improve the coordination of movements, to reduce spasticity, to support rehabilitation measures, to build up muscles, to maintain muscles or similar programs. Garments with a large number of stimulation electrodes require a control program in which the individual stimulation impulses are coordinated with each other. If two antagonistic muscle groups are activated simultaneously, this does not lead to movement of a limb and a training effect or support or equalisation of a movement, but to increased muscle tension and correspondingly unpleasant effects for the patient. The control of the stimulation is therefore very complex and also depends on the purpose and respective program to be completed. For this reason, the complex control programs are usually created once and stored in the respective control unit for a longer period of time. Adaptation to the respective patient and also to changes in the patient with regard to the success of the stimulation, for example due to muscle growth achieved through previous training units, a regularisation of movement sequences or the like, are not taken into account. The activation program in the proposed method activates, deactivates or modulates the respective stimulation electrodes on the basis of a basic program which, for example, defines the stimulation for various movement sequences for the upper or lower extremity. This basic program provides for certain parameters to be changed that affect the user of the garment. The parameters are individual and are entered, in particular after a prompt by the basic program and include, for example, the age of the user, a previous illness, a restriction in the ability to move, certain measured parameters such as body height, body weight, maximum angles of mobility of certain joints and the like. The basic program is automatically changed and an adapted stimulation program or training program is created depending on the at least one parameter that is entered as part of the stimulation electrode control system. This prevents, for example, excessive stimulation intensity or stimulation frequency being set or stimulation taking place that is contraindicated with regard to an illness or a current movement restriction.

In one embodiment, the stimulation program or training program is generated from several modified basic programs, so that a significantly more complex stimulation program or training program can be created for the respective user. For example, a first basic program can include stimulation of the muscle groups around the elbow joint, while a second basic program can include stimulation of the muscle groups around a shoulder joint. The training program is then created from an individually and automatically adapted combination of the two basic programs depending on the individual parameters entered in each case. The combination of the different basic programs can also lead to the activation programs being modified accordingly in order to exclude the possibility that, for example, a certain flexion or extension of the elbow joint is performed or not performed when the upper arm is in a certain position.

In one embodiment of the method, a parameter value range is specified for each parameter that can be set individually, within which the respective individual parameter should be located. This prevents stimulation outside the medically desired or intended ranges. The parameter value range that is specified for the respective parameter can also change with a combination of several basic programs or depending on other entered parameters, whereby the extent of the respective change or adjustment of the parameter value ranges takes place automatically.

In one embodiment, the control device has an HMI (human-machine interface) via which the at least one parameter is entered. The HMI can, for example, be a separate, special input device; alternatively, the HMI is a mobile end device, such as a tablet or mobile phone, on which an application runs that enables the orthopaedic technician or the person using the device to enter various parameters or feedback.

The at least one parameter, which is entered individually, is provided from a group containing age, physiological parameters, previous illnesses and individual perception. The group can contain further parameters.

In one embodiment of the method, at least one sensor is arranged on the item of clothing and is coupled to the control device, whereby the training program is or can be changed automatically on the basis of sensor values transmitted to the control device by the sensor attached to the item of clothing. The sensor can, for example, be designed as a force sensor, acceleration sensor, position sensor or a sensor for biosignals. Biosignals are, for example, temperature, humidity, myoelectric signals, skin conductivity, oxygen saturation or similar. The stimulation electrodes can also be designed as sensors for detecting myoelectric signals and fulfil both functions. In addition to the individual parameters, the stimulation or training program is automatically adapted on the basis of objectively measured values, for example to prevent undesired or incomplete movements, fatigue or other undesired effects, so that the adapted stimulation by the stimulation electrode can achieve correct execution of the training program and better training success.

In one embodiment, the control device is housed in an electronic unit that can be detachably attached to a docking station on the garment, whereby the activation program is transmitted to the control device in the electronic unit, in particular from an external database, before and/or after the stimulation program or training program is created. This makes it possible for general conditions for the stimulation program or training program to be stored in a database and transmitted externally to the control device in the electronic unit. This external, central data transmission has the advantage that new findings regarding the consequences of the training program or new medical findings can be incorporated into the design of the activation program without the need for time-consuming reprogramming on site. Alternatively, or additionally the activation program is transmitted after the stimulation program or training program is created.

In a further development, each garment is assigned an identifier which is transmitted to the control device. The identifier transmits information to the control device, e.g. about the number and/or location of the electrodes on the garment, so that the control device is informed at all times about the current status of the garment, which garment is connected to the control device and where which electrode is located on the garment. The respective stimulation program or training program is confirmed or adapted on the basis of this information, which ensures that all stimulation electrodes present and intended for the training program are controlled and that electrodes that are not present are not supplied with stimulation signals from the control device in vain.

In a further development, several garments with corresponding stimulation electrodes are coupled together, whereby the training programs in the control devices of the garments are coordinated with each other. A control device is provided for each item of clothing, which can be connected to the respective docking station via an electronic unit. The control units, for example for a shirt and a pair of trousers, communicate with each other as soon as the garments have been coupled, whereupon the stimulation programs or training programs in the respective garments are automatically adjusted. This means, for example, that muscle groups that are required for a movement that is performed by muscle groups that are not covered by just one item of clothing can be controlled accordingly. Complex movement sequences and movement programs in particular require the stimulation programs to be coordinated accordingly.

In a further development, after the stimulation program or training program has been created, a change request or confirmation request is generated in the control device or in the control devices, via which the automatically created training program or stimulation program is finally approved by the orthopaedic technician or, if applicable, by the user. This takes account of individual experience or individual perception and increases acceptance of the procedure and the use of the garment or garments. The query is made via the HMI, for example on the screen of a mobile device, and ends, for example, with the confirmation and approval of the modified basic program as a training program.

In a further development, sensor data, training data and/or input data are stored in the control device and/or transmitted to an external evaluation device. Stimulation electrodes can also be used to record biosignals, for example myoelectric signals, so that feedback is provided, particularly in conjunction with other sensor data, on how which stimulation works, which movement is actually performed by the person using the device and whether training goals and training progress are being achieved. Individual comments and subjective feedback from the patient or orthopaedic technician can also be entered and used as data for evaluation.

For the purpose to provide a connection that is as durable and reliable as possible and that can withstand high external stresses, an embodiment provides a docking station for fixing to a textile component or textile surface of a garment has a receiving frame for arrangement on a first side of the textile surface, wherein a printed circuit board is arranged in the receiving frame, on which printed circuit board conductor sections are arranged which are designed to penetrate the textile surface, with an anchor frame for arrangement on a second side opposite the first side of the textile surface, wherein the anchor frame can be fastened to the receiving frame via fastening elements penetrating the textile surface while receiving the textile surface. In one embodiment, guides are formed on the anchor frame to accommodate conductor sections and/or mechanical load-bearing elements such as solder sleeves, which are connected to the conductor sections. The mounting frame is advantageously arranged on the side of the textile surface facing away from the user's body. The holding frame serves to hold a printed circuit board so that a stable assignment of the printed circuit board to the holding frame can be achieved. The mounting frame supports and stabilises the printed circuit board, whereby conductor sections are arranged on the printed circuit board, which penetrate the textile surface when mounted. For this purpose, the conductor sections have a corresponding length that makes it possible to move the conductor sections through the textile surface. The conductor sections are, for example, wires, pins or other essentially dimensionally stable, in particular flexible, electrical conductors. The anchor frame is designed for arrangement on the second side of the textile surface facing the body and is positioned there in the assembled state. The anchor frame forms the counterpart to the receiving frame and is fixed by means of fastening elements that are arranged or formed on the receiving frame, so that the textile surface is penetrated by the fastening elements and is received and held between the anchor frame and the receiving frame. The mounting frame and the anchor frame are secured to the textile surface by the fastening elements.

In one embodiment, there are guides on the anchor frame for holding the conductor sections and/or mechanical load-bearing elements, for example solder sleeves, which are connected to the conductor sections, whereby the mechanical load-bearing elements are connected to the conductor sections of the printed circuit board. The mechanical load-bearing elements are used to absorb forces that are applied to the conductor sections from the outside and transfer these forces to the anchor frame. The mechanical load-bearing elements and conductor sections are guided, mechanically secured and fixed, at least orientated, via the guides in the anchor frame so that a defined transition is provided between the stable conductor sections and the flexible, in particular elastic, areas of the textile surface and elastic electrical conductors. Conductor sections and elastic conductors that are directly connected to each other can also be fixed and held in the guides. The conductor sections and the elastic conductors can be connected to each other with a material bond, for example by a block connection; alternatively or additionally, mechanical connection methods can be used to connect the conductor sections to the elastic conductors. The connection can be made using one or more clamps, screw connections, sleeves, sleeves, crimp connections, adhesives, hooks, loops and/or shrink sleeves.

In one embodiment, a cover, for example a lid, is attached to the anchor frame, in particular attached in a form-fitting and removable manner, so that on the second side, i.e. the side facing the body, there is a cover, in particular a closed cover of the anchor frame, so that unevenness or individual protrusions of conductor sections that protrude through the textile surface do not lead to unpleasant contact points with the patient's skin. In addition, the cover or lid provides mechanical protection as well as protection against moisture when the garment is worn or washed.

In one embodiment, the conductor sections, mechanical load-bearing elements and/or elastic conductors are clamped in the guides; in particular, the mechanical load-bearing elements, conductor sections and/or elastic conductors are clamped in the guides via the cover or the lid. Alternatively or additionally, the guides are designed in such a way that, in conjunction with the mechanical load-bearing elements, they effect a mechanical, in particular clamping, fixation within the guides. When the cover is placed on or fixed to the anchor frame, the cover together with the guides and the mechanical load-bearing elements can mechanically secure the latter to the anchor frame, in particular against tensile loads. Forces that are transmitted from the outside to the conductor sections via conductors that are attached to the conductor sections in a mechanical and force-transmitting manner are not passed on and transmitted to the printed circuit board. The elastic conductors are connected to electrodes and/or sensors and fixed to the garment and possibly the textile surface so that forces act on the elastic conductors when they are moved. These forces are absorbed in the anchor frame by fixing the mechanical load-bearing elements.

In one embodiment, the mechanical load-bearing elements surround a connection point of the conductor sections each with an elastic conductor coming from the electrodes and/or sensors on the garment in order to provide a protective effect against external influences in addition to a simplified fixing via a wrapping or sheathing, for example as a solder sleeve or shrink sleeve, in particular with seals. The external influences can be mechanical influences such as pressure or bending as well as chemical influences such as sweat, other body fluids or detergents. Within the mechanical load-bearing elements, the conductor sections and the elastic conductor are electrically conductively connected to each other, in particular mechanically connected to each other. A mechanical connection can also be an electrically conductive connection, for example by forming eyelets and hooks at the ends of the conductor sections and the elastic conductors and coupling them together.

In one embodiment, the fastening elements are formed or fixed on the mounting frame or on the anchor frame and are designed, for example, as form-fit elements that can be bent over or brought into engagement with a counterpart on the respective opposite component. The fastening elements can, for example, be bent or hooked and/or clamped into a counterpart in an elastic configuration.

In one embodiment, the printed circuit board is mechanically fixed in the mounting frame, for example clamped in place or friction-locked and/or material-locked using form-fit elements. Force-fit fastening can be achieved by pressing, for example, while a material-fit connection can be achieved by welding or bonding. All types of fastening can also be combined individually or together.

In one embodiment, the printed circuit board has contact areas on the side facing to and/or facing away from the textile surface. The contact areas are electrically conductively connected to the conductor sections so that these contact areas can contact the detachable controller or the detachable electronic unit, which can be attached to the mounting frame. The stable assignment of the contact areas of the printed circuit board to the mounting frame ensures secure and detachable contact between an electronic unit and the conductor sections and thus also with the electrical, elastic conductors attached to the conductor sections. Alternatively, or additionally, contact areas are arranged on the surface of the circuit board facing away from the textile surface to provide the possibility to establish an electric contact between an electronic unit or stimulation unit and the circuit board upon attaching the electronic unit or stimulation unit to the docking station.

In one embodiment, coupling devices are provided on the mounting frame for detachably securing an electronic unit, which can be designed as a stimulation device and/or evaluation device. The coupling devices enable the electronic unit to be detachably attached to the mounting frame so that the garment with the docking station can be washed without the electronic unit, for example. The coupling devices mechanically attach the electronic unit to the docking station and simultaneously connect the electronic unit to the circuit board via electrical contacts or another connection for transmitting the signals to and from the electrodes and/or sensors on the garment.

One embodiment relates to a garment with at least one docking station as described above, wherein the receiving frame and the anchor frame are arranged on opposite sides of the textile surface and are connected to one another via fastening elements penetrating the textile surface, wherein the conductor sections project through the textile surface and are connected to an elastic conductor on the side of the textile surface facing away from the printed circuit board. In one embodiment, the conductor sections and the elastic conductors are connected via mechanical load-bearing elements such as sleeves or solder sleeves, whereby the mechanical load-bearing elements are fixed in the guides of the anchor frame. Garments must be elastic to enable comfortable wearing behaviour and to hold the sensors or electrodes, which are attached to the inside of the garment, in the intended locations. The connection between the electrodes and/or the sensors on the garment is therefore made via elastic conductors, via which signals are transmitted from the sensors and/or electrodes to the docking station on the circuit board and from there to the electronic unit via the contact areas. To enable stable attachment of the docking station, which holds the electronic unit, to the garment, the textile surface is clamped as part of the garment between the holding frame and the anchor frame. For this purpose, fastening elements, which are arranged on the receiving frame or the anchor frame, are passed through the textile surface so that the receiving frame can be coupled to the anchor frame in a form-fit or force-fit manner. The conductor sections, which are attached to or formed on the printed circuit board, also penetrate the textile surface from the outside towards the inside of the textile surface, so that the ends of the conductor sections are located on the inside facing the body when the garment is put on. This means that the ends of the conductor sections are located on the side of the textile surface facing away from the printed circuit board. In one embodiment, the conductor sections are mechanically and electrically connected to the elastic conductors via the mechanical load-bearing elements, for example solder sleeves. The mechanical load-bearing elements themselves are fixed, in particular clamped, in the guides of the anchor frame and provide the stable localisation between the elastic conductors and the docking station.

In one embodiment, the anchor frame is covered by the cover so that the inner surface of the anchor frame is closed. In addition to a stable cover, the cover can also be made of a watertight, at least water-repellent but air-permeable fabric. The cover prevents moisture from penetrating into the anchor frame. The guides within the anchor frame are closed by the mechanical load-bearing element so that moisture or other contaminants cannot penetrate the anchor frame once it is covered by the cover.

In one embodiment, the cover clamps the mechanical load-bearing elements in the guide or guides, which also has a sealing effect in addition to a mechanical fixing effect. In one embodiment, the load-bearing elements also have seals or are made of a sealing material so that when the load-bearing elements are clamped in the guides via the cover or the lid, there is a passage for the electrical conductor in the area of the connection with the conductor sections that cannot be penetrated by moisture.

In one embodiment, the textile surface consists of an elastic material and is held stretched between the mounting frame and the anchor frame. The textile surface is made of a material that allows penetration through the fastening elements of the frame. The pre-tensioned textile surface within the frame prevents creasing during wear and keeps the textile surface smooth within the frame.

In one embodiment, a protective cover, in particular a protective textile cover, is attached to the anchor frame and the load-bearing elements on the textile surface to provide an inner surface that is as smooth as possible, without any heels, and to increase the wearing comfort of the garment. The protective cover also covers the lid and, if necessary, the ends of the holding elements such as sleeves, sockets, screw connections or the like that protrude from the anchor frame.

In a further embodiment, sensors and/or electrodes are arranged on the elastic conductors which are connected to the conductor ends, which in turn are attached to the garment so that they can pick up signals from the body of the user of the garment or can be stimulated. The signals picked up by the sensors or electrodes are, for example, biosignals of the human body, such as temperature, skin moisture, pulse, oxygen saturation or the like and/or other physical parameters such as forces, accelerations, positions, velocities and the like. The elastic conductors are, for example, embedded or sewn into the garment, in particular in textile areas of the garment.

In one embodiment the method for attaching a docking station to a textile surface of a garment, wherein the docking station comprises a receiving frame with a printed circuit board and conductor sections projecting therefrom and an anchor frame, provides for piercing the textile surface with the conductor sections of the printed circuit board attached to the receiving frame, connecting the mounting frame and the anchor frame via fastening elements that penetrate the textile surface, connecting the conductor sections with elastic conductors to create an electrically conductive connection and fixing the conductor sections to the anchor frame on the side of the textile surface facing away from the printed circuit board. This enables the docking station to be permanently attached to the garment, whereby the fixing, in particular pressing the conductor sections into or onto the anchor frame, makes it possible to absorb the tensile forces that occur from the elastic conductors when the garment or textile surface is used and to keep them away from the printed circuit board. The forces that occur from the elastic conductors during use of the garment with an electrical unit, for example a stimulation unit or a controller for receiving or emitting electrical signals, are absorbed by fixing the conductor sections to the anchor frame. There is no further conduction via the conductors to the circuit board, so that a secure and detachable contact between the electrical unit and the garment can be achieved via the docking station.

One embodiment provides that the conductor sections and the elastic conductors are each connected to one another via a mechanical load-bearing element, whereby the load-bearing element advantageously surrounds the connection point between the respective conductor section and the respective elastic conductor. For this purpose, the mechanical load-bearing element has, for example, a shrink sleeve that seals the connection area between the respective conductor section and the elastic conductor, so that a permanent and stable connection between the textile and an electrically conductive component is achieved. The docking station is connected to the textile surface of a garment in a force-decoupled, tension-resistant and wash-resistant manner so that no surfactants, acids or alkalis can cause corrosion at the contact point between the conductor sections and the elastic conductors, even when the garment is washed. In one embodiment, the mechanical load-bearing element is inserted into a guide in the anchor frame and fixed, in particular pressed. This enables simple and tight transmission of electrical signals without the risk of corrosion at the connection point between the electrical conductor and the conductor sections.

The anchor frame is positively or non-positively secured to the mounting frame via fastening elements arranged on the mounting frame or anchor frame. Form-fit fastening can be achieved, for example, by subsequent bending or via a springloaded clip connection, while force-fit fastening is achieved by clamping the anchor frame to the mounting frame via the fastening elements, which interact with corresponding counterparts.

In one embodiment, the textile surface is tensioned before the mounting frame is connected to the anchor frame to achieve a smooth surface within the area of the textile surface surrounded by the anchor frame and the mounting frame.

In one embodiment, a cover or lid is placed on the anchor frame and fastened to the anchor frame. The anchor frame is closed by the cover or lid; at the same time, the cover or lid can fix the conductor sections and/or the mechanical load-bearing element to the anchor frame, in particular in the guides formed or arranged on the anchor frame.

In one embodiment, the anchor frame, which is arranged in particular on the inside of the garment or textile surface facing a patient or user, is covered by a lamination, in particular a textile, covering the anchor frame, the lamination being fixed to the textile surface. This achieves a smooth and pleasant surface on the side of the textile surface with the anchor frame.

Various garments can be used to carry out electrostimulation therapies of this kind. Depending on the muscle group, muscle, nerve and/or nerve fiber to be stimulated, for example, pants, shirts or jackets or complete suits, such as overalls, can be used. Garments are also understood here to mean wide belts, bandages or harnesses made of belts and/or straps, which can be arranged on the wearer's body and fitted with electrodes and/or sensors. The number and position of the electrodes and/or sensors is decisive for which therapies and stimulations can be carried out. A disadvantage is that, although removable stimulation units and controllers are known which can be removed from the docking station when they are not needed, each individual controller and each individual stimulation device can only be used for a single garment.

An embodiment is therefore based on the task of proposing a system with which this disadvantage can be eliminated or at least alleviated.

The problem set is solved by a system for electrostimulation of at least one muscle, a muscle group, a nerve and/or a nerve fiber, the system comprising at least one garment having a docking station and a plurality of electrodes and/or sensors, and a stimulation unit which can be connected to the docking station in such a way that a mechanical and electronic connection is established between the stimulation unit and the docking station, wherein the stimulation unit comprises an electrical controller which is adapted to use a stimulation program from a first electronic data memory, depending on configuration data received via the electronic connection, to activate at least one electrode of the garment.

With such a system, it is possible to use a single stimulation unit for different garments and different arrangements of electrodes and/or sensors if the individual components of the system are suitably selected. The stimulation unit receives configuration data via the electronic connection to the docking station, the configuration data preferably contains the number, position and/or connection of the individual electrodes and/or sensors. Preferably, the configuration data contains all the information required to select a stimulation program from the first electronic data memory.

Preferably, this selection is made on the basis of the configuration data by the electrical control unit of the stimulation unit. The electrical control unit is preferably an electronic data processing device or has such a device. This has a processor which is set up to execute electronic data processing programs which it reads out from the first electronic data memory, for example. The electronic data processing device of the electrical control unit processes the configuration data received and can select the stimulation program to be used for the desired application, for example therapy, by means of a suitable data processing program.

In some advantageous embodiments, the first electronic data memory is part of the stimulation unit. In other advantageous embodiments, the first electronic data memory is not part of the system comprising the garment and the stimulation unit, but part of an external device, for example a computer or a mobile device, for example a smartphone. In this case, it is advantageous if the stimulation unit has a communication interface, preferably a wireless communication interface, via which it can communicate with the external device. In some embodiments the first electronic data memory is a cloud memory, which is accessible for the electrical control unit of the stimulation unit via an app which is used to control a communication device, which can also be called a communication interface, such as a WLAN or a Bluetooth interface. The electrical control unit is in this embodiment capable of downloading the stimulation program from the first electronic data memory.

Preferably, the system has a plurality of garments, each of which has a docking station and a plurality of electrodes and/or sensors. Preferably, not all garments are identical. The plurality of garments belonging to the system in this embodiment preferably differ at least in the number, the position and/or arrangement of the electrodes and/or sensors. Alternatively or additionally, they differ in the connection of the electrodes and/or the sensors to each other. Alternatively or additionally, the plurality of garments are garments that can be worn on different parts of a wearer's body. For example, a system can have at least one shirt and at least one pair of trousers. It is important that the docking stations of all garments of the system are designed in such a way that the stimulation unit of the system can be arranged on each of these docking stations. This means that it is no longer necessary to use different stimulation units for different garments and/or different therapies and stimulation methods.

In a preferred embodiment, the configuration data includes information about the garment and/or the number and/or the position and arrangement of the electrodes and/or the sensors of the respective garment. The information about the garment preferably includes information about the type of garment, for example about the part of the body on which the garment can be worn, and/or information about the material, for example a textile, from which the garment is made, and/or its mechanical, in particular flexible and elastic, properties and/or its electrical properties, for example its electrical conductivity. Alternatively or additionally, the information about the garment includes data about the temperature and/or the humidity of the garment.

Advantageously, each garment has a second electronic data memory in which the configuration data is at least partially, but preferably completely, stored. In addition, the garment preferably has a further electronic unit which is in communication with the electrical control of the stimulation unit when the stimulation unit is connected to the docking station. The further electronic unit of the garment then transmits the configuration data, insofar as they are stored in the second electronic data memory, to the electrical control unit of the stimulation unit. The further electronic unit can be a part of the docking station of the garment. In other embodiments, the further electronic unit is only connected to the docking station but is a separate element which is provided at some convenient position of the garment.

In order to establish the electronic connection between the stimulation unit and the docking station, there is an interface between the two, which can be in the form of a plug and a socket, for example. The plug or socket can be located on the stimulation unit and the socket or plug on the docking station. It is important that when the stimulation unit is connected to the docking station, a plug interacts with a socket and thus the electronic connection is established via the electrical contacts contained therein.

Preferably, the electrical control unit of the stimulation unit and/or the further electronic unit of the garment recognizes when the electronic connection has been established, so that the transmission of the configuration data is started automatically.

In a preferred embodiment, the stimulation unit has an energy source, in particular a current source and/or a voltage source, preferably a battery or a rechargeable battery.

The invention also solves the problem posed by a stimulation unit for a system of the type described here. The stimulation unit has an electrical controller which is set up to receive and process configuration data and to send stimulation pulses to at least one of the electrodes on the basis of the configuration data. Information about the stimulation pulses to be emitted, for example their duration, their strength and/or their number, is stored in the stimulation program, which is executed by the electrical controller of the stimulation unit after it has been selected on the basis of the configuration data. This information, which is read out from the stimulation program, is converted into electrode stimulation signals, which are then processed and lead to the stimulation pulses.

The invention also solves the problem posed by a method for controlling a system of the type described here. In the method, the following steps are carried out:
- Connecting the stimulation unit to the docking station of a garment so that a mechanical and electronic connection is established between the stimulation unit and the docking station,
- Receiving configuration data via the electronic connection by the electrical controller of the stimulation unit,
- Processing the received configuration data in the electrical control unit of the stimulation unit,
- Generating electrode stimulation signals on the basis of the processed configuration data, and
- Stimulating at least one electrode using the electrode stimulation signals.

Preferably, the electrical control of the stimulation unit receives the configuration data from the further electronic unit of the garment. It is not necessary, but advantageous, if the complete configuration data is transmitted from the further electronic unit of the garment to the electrical control unit of the stimulation unit.

In a preferred embodiment, the electrical control unit of the stimulation unit receives the configuration data from at least one sensor of the garment. Preferably, the electrical control unit of the stimulation unit sends a control signal to at least one sensor and/or at least one electrode, wherein the at least one sensor and/or the at least one electrode then send a sensor signal containing configuration data back to the electrical control unit in response. Particularly preferably, the electrical control system of the stimulation unit receives a portion of the configuration data from the second electronic data memory of the garment, which is preferably arranged in the docking station, and another portion of the configuration data from at least one sensor. Electrodes on the garment can also be used as sensors. For example, they can determine information about how good the electrical contact between an electrode and the wearer's skin is, what the temperature and/or moisture of the wearer's skin is and whether the electrical connection between the electrical control of the stimulation unit and/or the further electronic unit of the garment on the one hand and the respective electrode on the other is sufficient and functioning.

The corresponding control signal can be transmitted by the electrical control unit of the stimulation unit directly to the at least one sensor and/or the at least one electrode or can be transmitted by the electrical control unit of the stimulation unit to the electronics unit of the docking station, which processes this control signal and transmits corresponding control signals to the sensors and/or electrodes to be addressed. The sensors and/ or electrodes can transmit their sensor signals directly to the electrical control unit of the stimulation unit or transmit them to the electronics unit of the docking station, which transmits the sensor signals in unaltered or processed form to the electrical control unit of the stimulation unit.

Preferably, the further electronic unit of the garment recognizes when an electronic connection has been established between the docking station and the stimulation unit and then automatically sends the corresponding control signals to the sensors and/or electrodes. The further electronic unit of the garment can recognize the establishment of the electronic connection, for example, by the fact that the further electronic unit of the garment is connected to the energy source, for example the current source or the voltage source, of the stimulation unit. This is preferably used as a trigger for sending the control signals to the at least one sensor and/or the at least one electrode.

Such devices and systems might be not very flexible. The position of the individual electrodes cannot be subsequently changed, meaning that new devices have to be used if the existing electrode configuration turns out to be suboptimal or unusable. This makes the systems cost-intensive and inconvenient for the user, as different devices have to be used and may have to be adapted and manufactured again.

Therefore, an embodiment is based on the task of proposing a system with which these disadvantages can be eliminated or at least mitigated.

The embodiment solves the task set by a device for stimulating at least one muscle, muscle group, nerve or nerve fiber, wherein the device comprises a plurality of electrodes and/or sensors for placement on the skin of a user of the device and an electrical control unit and includes or is connectable to a power source, wherein over each muscle, muscle group, nerve and/or nerve fiber to be stimulated at least one electrode is positioned when the device is worn, which is characterized in that the electrical control unit is set up to stimulate a target muscle, a muscle group, a nerve and/or a nerve fibre by activating the electrodes in such a way that a current flow is caused between exactly one electrode over the target muscle, muscle group, nerve and/or nerve fibre and a plurality of other electrodes which act as reference electrodes.

The target muscle, the target nerve, the target muscle group and/or the target nerve fiber are hereinafter referred to as the target.

In the applied state of the device, an electrode is arranged above each target. By activating this electrode, it is therefore possible to generate a current flow that passes through the target. In contrast to prior art devices, this electrode is not assigned a unique counter electrode or reference electrode that receives the current flow. Instead, the electrical control unit is set up to use several electrodes as reference electrodes so that the current flow through the body of the wearer of the device can be controlled and conducted differently. This makes it possible, for example, to significantly reduce the current density compared to a single reference electrode, which is much more comfortable and less painful for the wearer. In addition, the current flow through the target can be better controlled. The current flows from the electrode positioned above the target, which can also be referred to as the target electrode, to the multiple reference electrodes. As these can be freely selected from the plurality of electrodes in the device, the current flow and current density can also be freely adjusted.

In a preferred embodiment, the electrical control unit is set up to activate the electrodes for stimulating the target in such a way that the current flow is generated between exactly one electrode above the target and all other electrodes, whereby these other electrodes act as reference electrodes and can therefore also be referred to as counter electrodes. This significantly reduces the current density, which makes using the device much more comfortable and pleasant for the wearer.

The electrical control preferably contains an electronic data processing device, for example a microprocessor, or is such a device. It has access to an electronic data memory in which preferably a program is stored which can be executed in the electronic data processing device and by means of which the electronic data processing device is able to determine from configuration data how the electrodes and/or sensors of the device should be activated in order to stimulate a predetermined target.

Preferably, electrical control is arranged to determine the number and selection of the reference electrode from configuration data containing information about the number and/or arrangement of the electrodes. Preferably, the configuration data also contains information about the target, i.e., the at least one muscle, the at least one muscle group, the at least one nerve and/or the at least one nerve fiber to be stimulated. In this way, no stimulation programs need to be provided to the electrical control unit so that it sends corresponding signals to the electrodes. Rather, the electrical control unit is informed, for example by reading from an electronic data memory, how many electrodes the device has, where these are located on the wearer's body when the device is worn and which electrode is the target electrode, i.e., the electrode located above the target.

In a preferred embodiment, the device has at least one sensor which is set up to record sensor data containing information about the effect of the stimulation, so that this can be read out, or at least determined from the sensor data. In a particularly preferred embodiment, the sensor data and/or the information contained therein about the effect of the stimulation is used to determine the number and selection of the reference electrode. Preferably, the electrical control unit is set up to trigger the sensor data after it has determined the target electrode and the reference electrode. For this purpose, the electrical control unit is set up to transmit control signals to the at least one sensor, which transmits sensor data in response to these control signals. This data can be transmitted directly to the electrical control unit or first transmitted to another unit, for example another electronic unit, which forwards it to the electrical control unit unchanged or in processed form. This sensor data can be used to determine the effectiveness of the stimulation so that a feedback loop can be set up. If the effectiveness of the stimulation falls below a predetermined limit value, the electrical control unit is set up to change the number and/or selection of the reference electrode. This allows the stimulation to be improved so that it is as effective as possible.

With a docking station and a plurality of electrodes and/or sensors. Particularly preferably, the device has a stimulation unit that can be connected to the docking station in such a way that a mechanical and electronic connection is created between the stimulation unit and the docking station.

All embodiments are part of the invention and can be combined with one another, whereby individual embodiments can be combined with one or more other embodiments and can be combined.

In the following, embodiments of the invention are explained in more detail with reference to the figures. They show:
Figure 1 - a system comprising a stimulation unit and a plurality of garments, each having a docking station,
Figures 2 to 4 - a mounting frame, a printed circuit board and an anchor frame of a docking station,
Figures 5 and 6 - various steps in the manufacture of the docking station,
Figure 7 - a cover of a docking station,
Figure 7A - a detailed view of the circuit board,
Figure 8 - an assembled docking station,
Figure 9 - a schematic sequence of a procedure for setting up a control system for the electrodes of the system shown in Figure 1, and
Figures 10 and 11- schematic representations of activated electrodes in such a system.

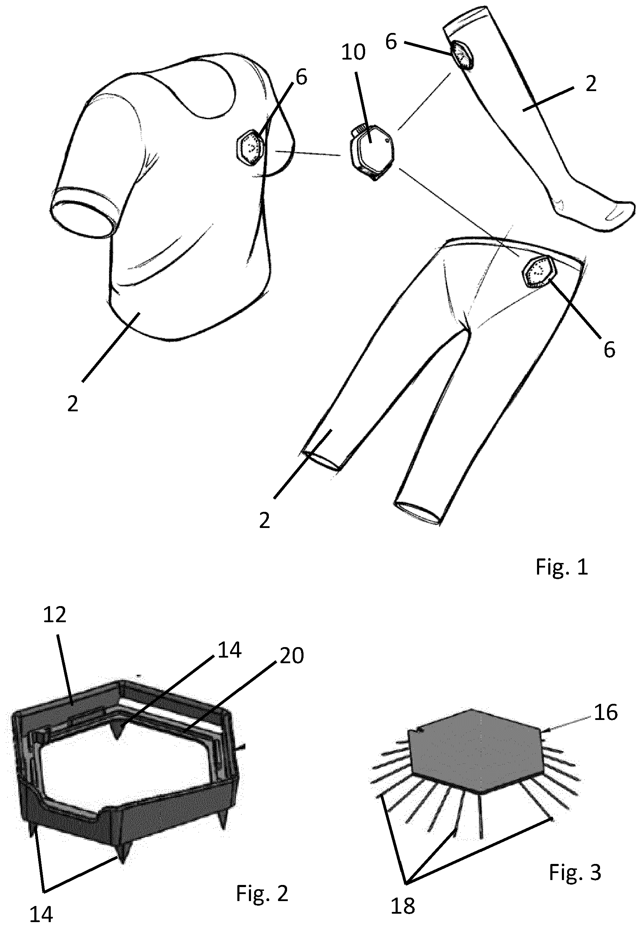

Figure 1 shows three garments 2, one of which is a T-shirt, one a pair of trousers and one a sock. They all have electrodes 4, which are not shown and which are in contact with the wearer's skin when the respective garment 2 is put on. Each of the garments 2 also has a docking station 6, which is connected to the electrodes 4, which are not shown, via elastic conductors 8, which are also not shown. The system shown in Figure 1 has an electronic unit 10 in form of a stimulation unit 10 that can be inserted into each of the docking stations 6, so that both a mechanical and an electronic connection is created between the docking station 6 and the stimulation unit 10. The stimulation unit 10, which is also referred to as the electronic unit 10, has an electrical controller that is set up to execute a training program and thereby activate electrodes 4 when the stimulation unit 10 is connected to the docking station 6 and the mechanical and electronic connection is established. Such a training program is also referred to as a stimulation program.

Figures 2 to 4 show different elements of the docking station 6 that are used to attach the docking station 6 to the respective garment 2. Figure 2 shows a receiving frame 12 which is arranged on a first side of a textile surface of the garment 2, the first side of the textile surface facing away from the body of the wearer when the garment 2 is put on. Fastening elements 14 are arranged on the lower side of the receiving frame 12 in Figure 2, which are arranged to be pierced through the textile surface of the garment 2 in order to mount the docking station 6 and the receiving frame 12.

Figure 3 shows a printed circuit board 16 on which a plurality of conductor sections 18 is arranged, which protrude beyond the outer edge of the printed circuit board 16.

When the docking station 6 is mounted, the printed circuit board 16 is positioned within the receiving frame 12. The mounting space has a support ledge 20 on which the printed circuit board 16 is positioned. When the docking station 10 is mounted, the conductor sections 18 penetrate the textile surface of the garment 2 and thus, like the fastening elements 14 of the receiving frame 12, extend to the second side of the textile surface, which faces the wearer's body when the garment 2 is in the mounted state.

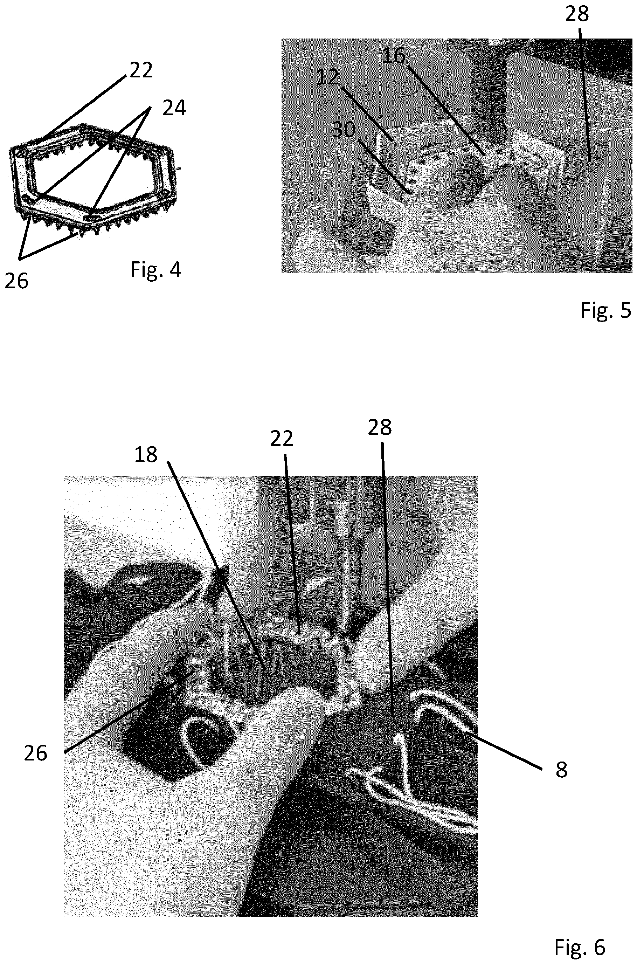

An anchor frame 22, which is shown in Figure 4, is arranged on this second side of the textile surface of the garment 2. On its upper side, which faces the textile surface in the assembled state, it has positive-locking elements 24 corresponding to the fastening elements 14 of the receiving frame 12, which are formed as recesses in the embodiment example shown. Guides 26 are provided on the opposite underside of the anchor frame 22, which are designed to accommodate conductor sections 18 or load-bearing elements connected to conductor sections 18.

Figure 5 shows a work step in the assembly of a docking station 6. The receiving frame 12 has been arranged on a textile surface 28 of a garment 2 not shown in such a way that its fastening elements 14 face the textile surface 28. The printed circuit board 16 has already been placed on the support ledge 20 of the receiving frame 12 in Figure 5. The conductor sections 18 protruding from the printed circuit board 16 cannot be seen in the state shown in Figure 5. Several solder joints 30, with which the conductor sections 18 are connected to the printed circuit board 16, are visible.

Figure 6 shows the situation on the opposite second side of the textile surface 28.

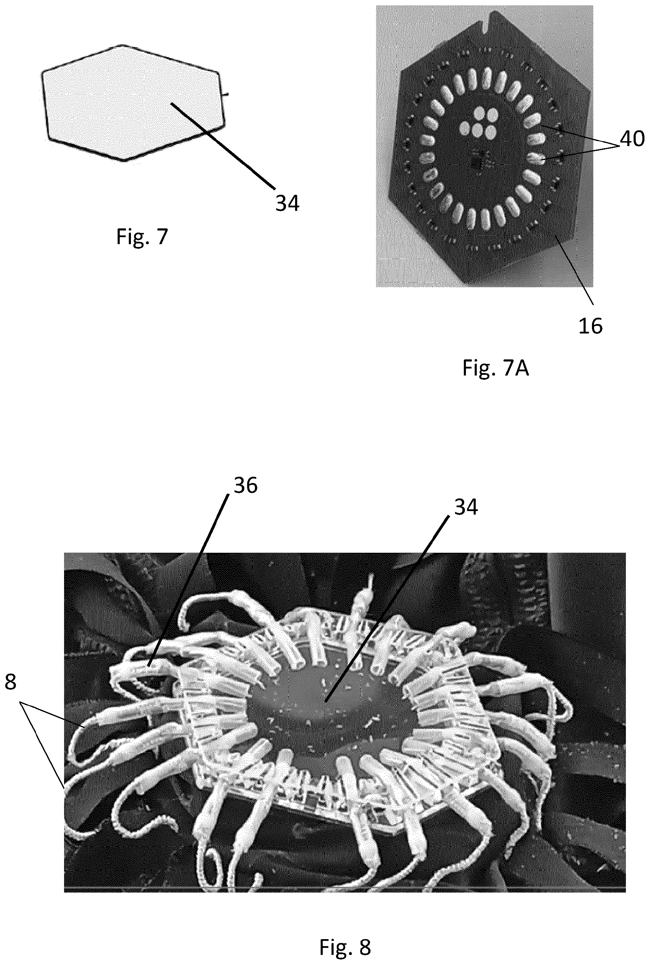

The anchor frame 22 is in contact with the textile surface 28 with its contact side, which is shown at the top in Figure 4. The guides 26 and the conductor sections 18, which protrude through the textile surface 28 inside the anchor frame 22, can be seen. The textile surface 28 has elastic conductors 8 which are connected to the conductor sections 18, thereby establishing an electrical connection between the electrodes 4 and the docking station 6. Once the elastic conductors 8 are connected to the conductor sections 18, the anchor frame 22 is covered by a cover 34, which is shown in Figure 7.

Figure 7A shows a printed circuit board 16 in detail with contact areas 40 on the surface of the circuit board 16 facing to the textile surface 28 when mounted into the receiving frame 12. Alternatively, or additionally, contact areas are arranged on the surface of the circuit board 16 facing away from the textile surface 28 to provide the possibility to establish an electric contact between an electronic unit 10 or stimulation unit and the circuit board 16 upon attaching the electronic unit 10 or stimulation unit 10 to the docking station 6. In the embodiment shown, the contact areas 40 are arranged in a circle and can be brought in direct contact with corresponding contacts of the electronic unit 10 or stimulation unit 10. By direct contact between contact areas of the stimulation unit 10 and the contact areas 40 of the circuit board, sensor data and or stimulation impulses can be transferred from the stimulation unit 10 to the sensors or electrodes 38, which are connected to the circuit board 16 via the elastic conductors 8.

Figure 8 shows the covered anchor frame 22, through the guides 26 of which pass load-bearing elements 36, which are designed, for example, as solder sleeves. They have an inner opening and an outer opening. A conductor section 18 is inserted into the load-bearing element 36 through the inner opening, which is located underneath the cover 34. An elastic conductor 8 is inserted into the load-bearing element 36 through the outer opening, which is located outside the anchor frame 22. The elastic conductor 8 is electrically connected to the conductor section 18 inside the load-bearing element 36. The load-bearing element 36 is clamped in the guide 26 of the anchor frame 22, which is also closed by the cover 34, in such a way that a tensile force acting on the elastic conductor 8 is not transmitted to the printed circuit board 16 via the conductor section 18.

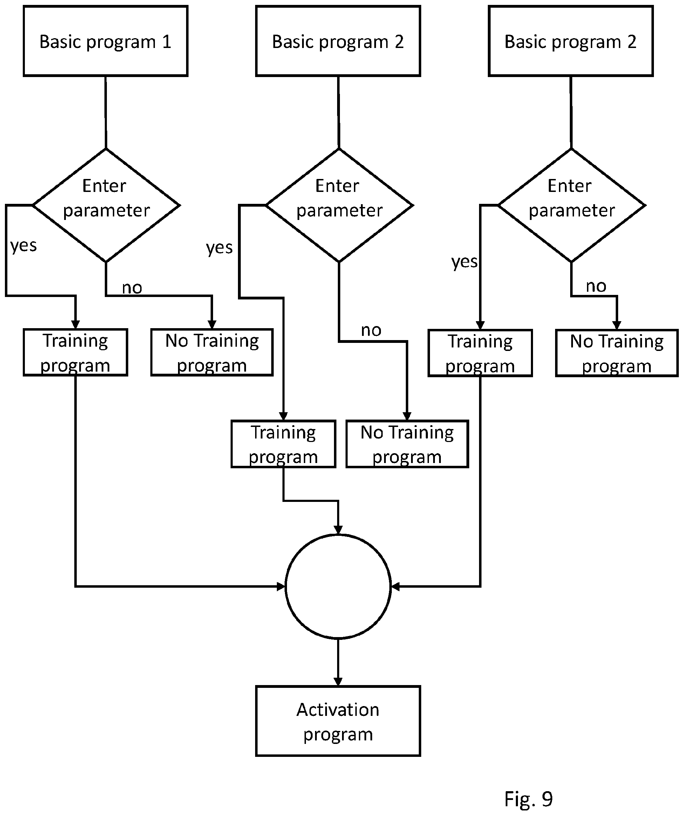

Figure 9 schematically shows a method for setting up a control system for stimulation electrodes of a system, for example according to Figure 1. Three basic programs are stored in a control device, which can be located outside the system, for example in the form of an external device such as a cell phone, laptop or computer or in the form of a cloud, the different basic programs are to be used in different therapies for different requirements. For example, it is possible to use basic program 1 for chronic pain, basic program 2 for spasticity and basic program 3 for other requirements. The basic programs form a basis, but they are not tailored to the individual patient. Additional parameters relating to the user of the garment must therefore be entered. This can be, for example, weight, gender or age. If no data is entered, no training program is created. This is the case for all three basic programs.

However, if parameters relating to the user of the garment are entered, these are used to generate a training program from the respective basic program that is individually tailored to the respective patient. The training program created in this way is transferred to the stimulation unit 10 of the system shown in Figure 1, for example, via an activation program and executed there.

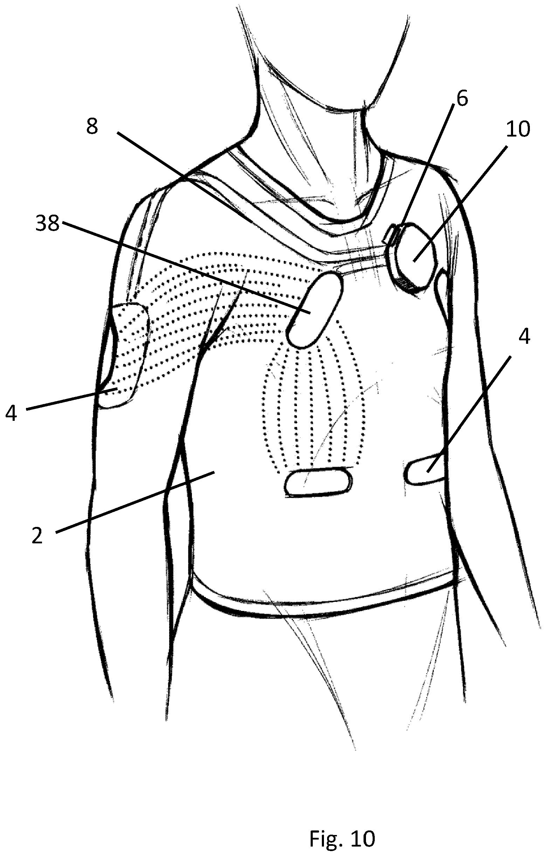

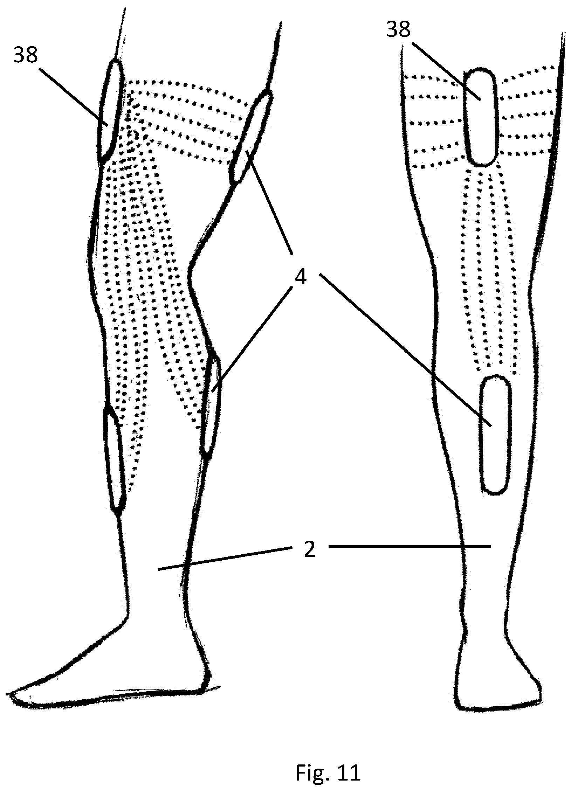

Figures 10 and 11 schematically illustrate the results of such a training program. In Figure 10, the garment 2 is in the form of a shirt and lies against the wearer's upper body. The stimulation unit 10, which has been already explained is also referred to as electronic unit 10, is positioned in the docking station 6. The garment 2 has several electrodes 4, which are positioned at different points on the garment 2 and therefore also at different points on the wearer's body. Elastic conductors 8 run inside the garment 2, only a few of which are shown for the sake of clarity. They are connected to the conductor sections 18 of the docking station 6, which are not shown, and thus provide an electrical connection between the electrodes 4 and the stimulation unit 10.

It goes without saying that all electrodes 4 are connected to the docking station 6 via such conductors, even if not every one of these connections is shown in Figure 10. It can be seen that one of the electrodes is activated as the target electrode 38.

Activation of the target electrode 38 causes a current to flow through the body and in particular through the muscle identified as the target, which can be controlled by selecting corresponding reference electrodes, which may also be referred to as counter electrodes. In this case, the current flow is shown by dotted lines, so that it can be seen that two reference electrodes are present.

In Figure 11, the garment 2 is a stocking that covers and envelops the entire leg. Figure 11 shows the same leg of a wearer from two different views. Figure 11 shows a frontal view on the right and a lateral view on the left. The garment 2 has several electrodes 4, which are used to stimulate a target muscle, in this case a thigh muscle. The target electrode 38 is located on the front of the thigh. The electrode 4 arranged on the back of the thigh and the two electrodes 4 positioned on the calf serve as reference electrodes.

### List of reference symbols

2 Garment
4 Electrode
6 Docking station
8 Elastic conductor
10 Stimulation unit/electronic unit
12 Receiving frame
14 Fastening element
16 Printed circuit board
18 Conductor section
20 Support ledge
22 Anchor frame
24 Positive locking element
26 Guide
28 Textile surface
30 Solder joint
34 Cover
36 Load-bearing element
38 Target electrode
40 Contact area

## Claims

1. Method for setting up a control system for stimulation electrodes (4) which are arranged on at least one garment (2) with at least one textile component (28) and are coupled to a control device in which an activation program is stored, with which it is determined when and which stimulation electrode (4) is electrically activated, deactivated or modulated, **characterised in that** at least one basic program is stored in the activation program, **in that** at least one parameter individual to the user of the garment (2) is entered for each basic program and **in that** the basic program is automatically changed and a stimulation program or training program is created as a function of the at least one parameter.

2. Method according to claim 1, **characterised in that** the stimulation program or training program is generated from several modified basic programs.

3. Method according to claim 1 or 2, **characterised in that** a parameter value range is specified for each parameter, within which the respective parameter is to be located.

4. Method according to one of the preceding claims, **characterised in that** the control device has an HMI (human machine interface) via which the at least one parameter is entered.

5. Method according to one of the preceding claims, **characterised in that** the at least one parameter is provided from a group comprising age, physiological parameters, previous illnesses, and individual sensation.

6. Method according to one of the preceding claims, **characterised in that** at least one sensor is arranged on the garment (2), which sensor is coupled to the control device, and **in that** the stimulation program or training program is changed on the basis of sensor values transmitted to the control device.

7. Method according to one of the preceding claims, **characterised in that** the control device is arranged in an electronic unit (10) that can be detachably attached to a docking station (6) on the garment (2) and the activation program is transmitted from an external database before and/or after the stimulation program or training program is created.

8. Method according to one of the preceding claims, **characterised in that** each garment has an identifier which is transmitted to the control device.

9. Method according to claim 8, **characterised in that** information about the number and/or location of the electrodes (4) on the garment (2) is transmitted with the identifier of the control device and the stimulation program or training program is confirmed or adapted on the basis of this information.

10. Method according to one of the preceding claims, **characterised in that** several garments (2) are coupled together and the stimulation programs or training programs in the control devices of the garments (2) are coordinated with one another.

11. Method according to one of the preceding claims, **characterised in that** a change or confirmation query is generated in the control device after the stimulation program or training program has been created.

12. Method according to one of the preceding claims, **characterised in that** sensor data, training data and/or input data are stored in the control device and/or transmitted to an external evaluation device.
